# EUROPEAN PATENT SPECIFICATION

(11) **EP 3 708 193 B1**
(45) Date of publication and mention of the grant of the patent: **22.02.2023**
(21) Application number: 20172174.3
(22) Date of filing: 21.07.2016
(51) Int. Cl.: A61L 15/60, A01N 25/02, A01N 59/16, A61L 15/44

(54) **ABSORBENT ANTIMICROBIAL WOUND DRESSINGS**
ABSORBIERENDE ANTIMIKROBIELLE WUNDVERBÄNDE
PANSEMENTS POUR PLAIES ANTIMICROBIENS ABSORBANTS

(30) Priority: 24.07.2015 EP 15178270; 24.07.2015 US 201514807989
(43) Date of publication of application: 16.09.2020
(62) Divisional of application: 16741319.4
(73) Proprietor: Mölnlycke Health Care AB, 402 52 Göteborg (SE)
(72) Inventor: CARLSSON, Erik, 411 29 Göteborg (SE); HANSSON, Dennis, 424 56 Göteborg (SE)
(74) Representative: Tostmann, Holger Carl

(56) References cited:
- EP-A1- 1 884 253
- EP-A1- 2 055 327
- WO-A1-01/47569
- WO-A1-02/43743
- WO-A1-2015/022340
- WO-A1-2015/050682
- WO-A2-2012/118975
- GB-A- 2 382 305
- US-A- 4 885 855

## Description

### BACKGROUND OF THE INVENTION

Wound dressings have been used for centuries to promote healing, to protect damaged tissue from contamination by dirt and foreign substances, and to protect against infection. Studies have shown that a moist environment helps to promote wound healing. This has prompted the development of absorbent wound dressings that absorb and retain exudate. In order to further prevent infection and accelerate healing of wounds It would be desirable to Include antimicrobial agents in such absorbent wound dressings. It would also be desirable to do so in such a way that the antimicrobial agents are uniformly distributed within the dressing substrate. It would also be desirable that the antimicrobial agents are stabilized against degradation over time.

WO2015050682 discloses coating compositions comprising amphiphilic polymers, antimicrobial agents, solvents and optionally water.

EP2055327 discloses lubricant coating compositions comprising isocyanate terminated prepolymers, hydrophilic polymers, antimicrobial agents and ethanol.

### SUMMARY OF THE INVENTION

The invention is defined by the claims. Any subject matter falling outside the scope of the claims is provided for information purposes only.

The present invention stems In part from the recognition that certain antimicrobial agents (e.g., silver based antimicrobial agents) are only soluble In highly aqueous solutions while polymers useful for the production of absorbent wound dressings are Incompatible with highly aqueous solutions because they gel In the presence of water. Applying an antimicrobial agent which is only soluble In highly aqueous solutions to an absorbent wound dressing that is incompatible with highly aqueous solutions therefore poses a real challenge which has so far limited the development of absorbent antimicrobial wound dressings.

The present invention solves these and other problems by mixing the antimicrobial agent in a solvent system that comprises a non-aqueous solvent and one or more polymers. While not wishing to be bound by theory, the non-aqueous solvent is thought to avoid or reduce the amount of absorption that takes place during application of the antimicrobial agent while the one or more polymers are thought to help disperse the antimicrobial agent and avoid settling thereof. Thus, in one aspect, the present invention provides methods for preparing an absorbent antimicrobial wound dressing which comprise steps of (a) preparing an antimicrobial coating composition by mixing an antimicrobial agent and one or more polymers in a solvent system that Includes a non-aqueous solvent, wherein the one or more polymers in the antimicrobial coating composition are selected from the group consisting of cellulosic polymers, neutral poly(meth)acrylate esters, polyvinylpyrrolidone, polyvinylpolypyrrolidone, and combinations thereof, wherein the antimicrobial coating composition comprises 1-20% w/w of water, (b) contacting the antimicrobial coating composition of step (a) with a wound dressing substrate which comprises absorbent fibers, wherein the wound dressing substrate comprises cross-linked absorbent fibers, wherein the cross-linked absorbent fibers are capable of forming a swollen coherent gel upon absorbing a liquid, and (c) drying the product of step (b). The present disclosure also provides absorbent antimicrobial wound dressings including those prepared by these methods as well as antimicrobial coating compositions and methods of producing antimicrobial coating compositions.

As discussed below, in some embodiments, the one or more polymers in the antimicrobial coating on the wound dressing substrate modulate the rate at which the antimicrobial agent is released from the resulting wound dressing. Thus, In some embodiments, the rate of release of the antimicrobial agent from the wound dressing can be fine-tuned by varying the amount of the one or more polymers that are mixed with the antimicrobial agent and/or by using different polymers, polymers of different molecular weights or different polymer combinations. While the methods of the present Invention are particularly useful for antimicrobial agents that are only soluble in highly aqueous solutions (e.g., silver based antimicrobial agents) the ability to control the release rate of antimicrobial agents means that the methods can also be useful with antimicrobial agents that are soluble In non-aqueous solvents (e.g., PHMB). A further advantage of the present Invention is that, in some embodiments, wound dressings prepared according the methods described herein, particularly those involving silver based antimicrobial agents, have been shown to experience less discoloration over time compared to existing antimicrobial wound dressings. Another advantage of the present invention is that the antimicrobial coating compositions described herein also serve to hydrophilize the wound dressing substrates they are applied to, rendering a separate hydrophilization step unnecessary.

### BRIEF DESCRIPTION OF THE DRAWINGS

**Figure 1** depicts effects of changing the concentration of HPC on the amount of silver released from a wound dressing over time according to some embodiments of the present invention.

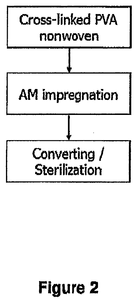

**Figure 2** depicts steps in a process for providing an antimicrobial (AM) wound dressing according to some embodiments of the present invention.

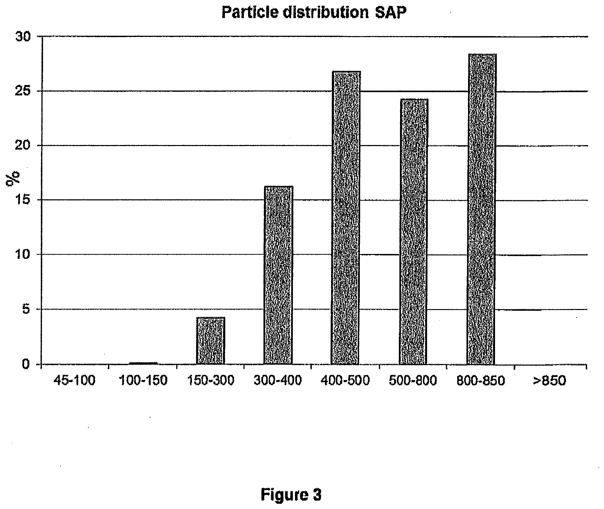

**Figure 3** depicts particle size distribution (values in µm) of superabsorbent particles (SAP) useful in the preparation of wound dressings according to some embodiments of the present invention.
**Figure 4** depicts discoloration of wound dressing substrate over time according to some embodiments of the present disclosure.
**Figure 5A** is a scanning electron micrograph image (LSEI detector at 1000x magnification, 30 Pa pressure) of polyvinyl alcohol fibers coated with a silver coating Including silver sulfate and hydroxypropylcellulose (HPC) (Prototype 7) according to the methods of the present invention. In this image, the silver particles are encapsulated by HPC in the silver coating, and the silver coating is clearly seen coating the fibers, most evidently at the junctions where fibers cross each other.
**Figure 5B** is a scanning electron micrograph image (BEC detector at 1000x magnification, 27 Pa pressure) of polyvinyl alcohol fibers coated with a sliver coating Including silver sulfate and hydroxypropylcellulose (HPC) (Prototype 8) according to the methods of the present Invention. The image shows a cross-sectional view of the fibers, wherein silver particles are seen in white. As can be seen no silver particles are present within the fibers.
**Figure 6A** Is a cross-section of a simple wound dressing according to one embodiment of the present disclosure.
**Figure 6B** is a cross-section of an island dressing-type wound dressing according to one embodiment of the present disclosure.
**Figure 7A** is a cross-section of a border dressing-type wound dressing according to one embodiment of the present disclosure.
**Figure 7B** is a cross-section of a wound dressing according to one embodiment of the present disclosure.

### DETAILED DESCRIPTION OF CERTAIN EMBODIMENTS

In general, the present invention provides methods for preparing absorbent antimicrobial wound dressings and antimicrobial coating compositions used in these methods. Disclosed are absorbent antimicrobial wound dressings produced by these methods.

### I. Methods for preparing an absorbent antimicrobial wound dressing

In one aspect, the present invention provides methods for preparing an absorbent antimicrobial wound dressing which comprise steps of (a) preparing an antimicrobial coating composition by mixing an antimicrobial agent and one or more polymers in a solvent system that comprises a non-aqueous solvent, wherein the one or more polymers in the antimicrobial coating composition are selected from the group consisting of cellulosic polymers, neutral poly(meth)acrylate esters, polyvinylpyrrolidone, polyvinylpolypyrrolidone, and combinations thereof, wherein the antimicrobial coating composition comprises 1-20% w/w of water, (b) contacting the antimicrobial coating composition of step (a) with a wound dressing substrate which comprises absorbent fibers wherein the wound dressing substrate comprises cross-linked absorbent fibers, wherein the cross-linked absorbent fibers are capable of forming a swollen coherent gel upon absorbing a liquid, and (c) drying the product of step (b).

In some embodiments, contacting step (b) Is performed using slot die, foulard, or kiss coating. In some embodiments, contacting step (b) is performed using slot die coating. In some embodiments, contacting step (b) is performed using foulard coating. In some embodiments, contacting step (b) is performed using kiss coating.

In some embodiments, drying step (c) is performed by passing the wound dressing substrate through a hot air convection oven or over hot plates. In some embodiments, drying step (c) Is performed by passing the wound dressing substrate through a hot air convection oven. In some embodiments, drying step (c) is performed by passing the wound dressing substrate over hot plates.

In some embodiments, the method further comprises a step of subjecting the wound dressing substrate to ethylene oxide sterilization after it has been dried In step (c). In some embodiments, the ethylene oxide sterilization is performed according to standard method ISO 11135-1, ISOITS 11135-2:2008, or ISO 11135:2014.

Suitable wound dressings (including suitable substrates and absorbent fibers or absorbent particles) as well as suitable antimicrobial agents, polymers, and non-aqueous solvents include those described below.

### A. Wound Dressings

### Substrate

The wound dressing substrate may comprise absorbent fibers, absorbent particles or a combination thereof. In some embodiments of the invention, the wound dressing substrate has a free swell absorptive capacity, corresponding to the maximum absorptive capacity of the wound dressing substrate, of at least 1 times its own weight as measured by EN 13726-1:2002 ("Free swell absorptive capacity"). For example, in some embodiments, the wound dressing substrate has a free swell absorptive capacity, corresponding to the maximum absorptive capacity of the wound dressing substrate, of at least 3 times Its own weight as measured by EN 13726-1:2002. For example, in some embodiments, the wound dressing substrate has a free swell absorptive capacity, corresponding to the maximum absorptive capacity of the wound dressing substrate, of at least 5 times Its own weight as measured by EN 13726-1:2002. For example, In some embodiments, the wound dressing substrate has a free swell absorptive capacity, corresponding to the maximum absorptive capacity of the wound dressing substrate, of at least 10 times Its own weight as measured by EN 13726-1:2002. For example, in some embodiments, the wound dressing substrate has a free swell absorptive capacity, corresponding to the maximum absorptive capacity of the wound dressing substrate, of at least 15 times Its own weight as measured by EN 13726-1:2002. For example, in some embodiments, the wound dressing substrate has a free swell absorptive capacity, corresponding to the maximum absorptive capacity of the wound dressing substrate, of at least 20 times Its own weight as measured by EN 13726-1:2002. For example, in some embodiments, the wound dressing substrate has a free swell absorptive capacity, corresponding to the maximum absorptive capacity of the wound dressing substrate, of at least 25 times its own weight as measured by EN 13726-1:2002. In some embodiments, the substrate comprises absorbent fibers. In some embodiments the absorbent fibers are in the form of a non-woven material. In some embodiments, the substrate comprises absorbent particles. In some embodiments, the absorbent particles are dispersed within a foam (e.g., without limitation, a polyurethane foam). In some embodiments, the substrate also includes non-absorbent fibers. In some embodiments, the absorbent fibers and/or absorbent particles are airlaid by spraying, needling, or carding together with non-absorbent fibers.

In some embodiments, the absorbent fibers or absorbent particles comprise a polymer. Without limitation, suitable polymers include polyvinyl alcohol, polysaccharides, polyacrylic acid, polymethacrylic acid, and copolymers comprising two or more monomers selected from vinyl alcohol, acrylic acid, and methacrylic acid. In general, It is to be understood that any reference herein to a polymer or monomer also encompasses salts of these polymers or monomers. In some embodiments, the absorbent fibers or absorbent particles comprise polyvinyl alcohol. In some embodiments, the substrate comprises absorbent fibers comprising polyvinyl alcohol. For example, in some embodiments the absorbent fibers may comprise a plurality of fibers comprising polyvinyl alcohol, such as the plurality of fibers disclosed in US 2013/0323195 and/or US 2013/0274415. In some embodiments, the absorbent fibers or absorbent particles comprise polyacrylic acid. In some embodiments, the absorbent fibers or absorbent particles comprise polymethacrylic acid. In some embodiments, the absorbent fibers or absorbent particles comprise a copolymer comprising two or more monomers selected from vinyl alcohol, acrylic acid, and methacrylic acid. In some embodiments, the absorbent fibers or absorbent particles comprise polysaccharides. In some embodiments, the polysaccharides are selected from the group consisting of cellulosic polymers, alginates, alginic acid, amylopectins, amyloses, beta-glucans, carrageenan, chitosans, gellan gums, gelatins, pectic acid, pectin, and xanthan gum. In some embodiments, the absorbent fibers or absorbent particles comprise a cellulosic polymer. In some embodiments, the absorbent fibers or absorbent particles comprise carboxymethyl cellulose. In some embodiments, the absorbent particles are superabsorbent particles. As used herein, the term "superabsorbent particles" denotes particles which can absorb at least 10 times their own weight in distilled water. In some embodiments, the superabsorbent particles comprise poly-N-vinylpyrrolidone, polyvinyltoluene sulfonate, polysulfoethylacrylate, poly-2-hydroxyethyl acrylate, polyvinylmethyloxazolidinone, polyacrylamide, polyacrylic acid, polymethacrylic acid, or copolymers or terpolymers of polysaccharides, polyacrylic acid, polyacrylamide, or polymethacrylic acid. In some embodiments, the superabsorbent particles comprise polyacrylic acid.

In some embodiments, the absorbent fibers or absorbent particles comprise a polymer that is cross-linked. In some embodiments, the absorbent fibers or absorbent particles comprise cross-linked polyvinyl alcohol. In some embodiments the substrate comprises absorbent fibers comprising cross-linked polyvinyl alcohol. In some embodiments, the absorbent fibers or absorbent particles are cross-linked by heat or chemical treatment. In some embodiments, the absorbent fibers or absorbent particles are cross-linked by heat. In some embodiment the wound dressing substrate may comprise cross-linked absorbent fibers, wherein the cross-linked absorbent fibers are capable of forming a swollen coherent gel upon absorbing a liquid. Thereby, the wound dressing substrate can be removed coherently from a wound. In some embodiments, the wound dressing substrate in a wet state having absorbed a maximum amount of 0.9 % by weight aqueous saline solution according to the "Free swell absorptive capacity method" (EN 13726-1), has a tensile strength of at least 0.2 N/ 2cm as measured by EN 29073-3:1992 (as applied to a 20 mm wide test piece). "For example, in some embodiments, the wound dressing substrate in a wet state has a tensile strength of at least 0.4 N/ 2cm such as at least 0.6 N/ 2cm or at least 0.8 N/ 2cm or at least 1.0 N/ 2cm, as measured by EN 29073-3:1992. In some embodiments, the wound dressing substrate in a wet state has a tensile strength of at least 2 N/ 2cm, for example at least 2.5 N/ 2cm, as measured by EN 29073-3:1992. In some embodiments, the wound dressing substrate in a wet state has a tensile strength of at least 3 N/ 2cm, for example at least 3.5 N/ 2cm. In some embodiments, the wound dressing substrate in a wet state has a tensile strength of at least 4 N/ 2cm, for example at least 4.5 N/ 2cm such as at least 5 N/ 2cm or at least 6 N/ 2cm or at least 7 N/ 2cm or at least 8 N/ 2cm or at least 9 N/ 2cm, as measured by EN 29073-3:1992. In some embodiments, the wound dressing substrate in a wet state has a tensile strength of at least 10 N/ 2cm, for example at least 15 N/ 2cm such as at least 20 N/ 2cm or at least 25 N/ 2cm, as measured by EN 29073-3:1992. In some embodiments, the wound dressing substrate in a wet state has a tensile strength of form 0.2 to 15 N/ 2cm as measured by EN 29073-3:1992. In some embodiments, the wound dressing substrate in a wet state has a tensile strength of form 0.2 to 10 N/ 2cm as measured by EN 29073-3:1992. In some embodiments, the wound dressing substrate In a wet state has a tensile strength of form 0.2 to 5 N/ 2cm as measured by EN 29073-3:1992. In some embodiments, the wound dressing substrate in a wet state has a tensile strength of from 1 to 4 N/ 2cm as measured by EN 29073-3:1992. As used herein, the term "wound dressing substrate in a wet state", should be understood as a wound dressing substrate which has been wetted to maximum absorptive capacity according to EN 13726-1:2002 ("Free swell" method). Thus, the tensile strength as given herein refers to the tensile strength as measured on such wet wound dressing substrate.

### Other Components

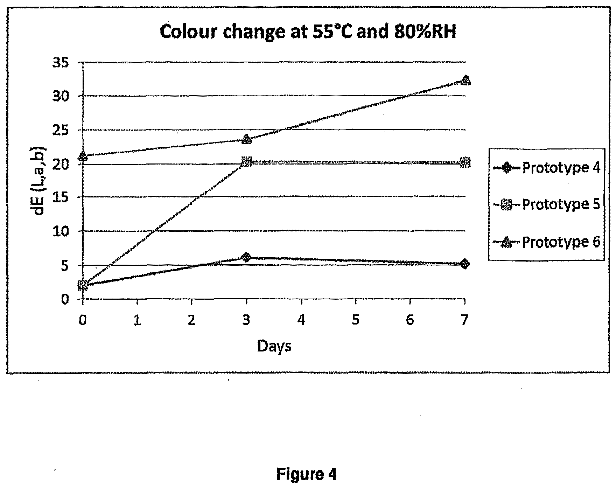

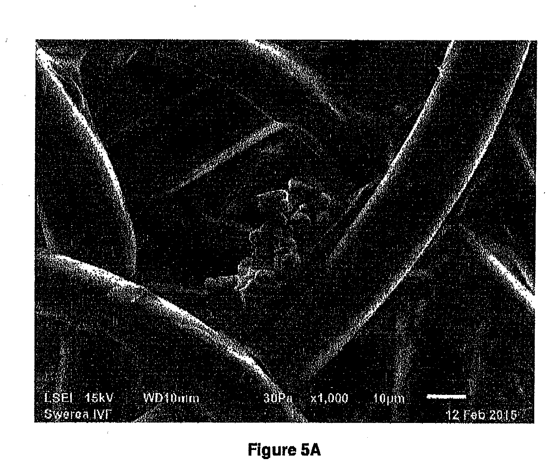

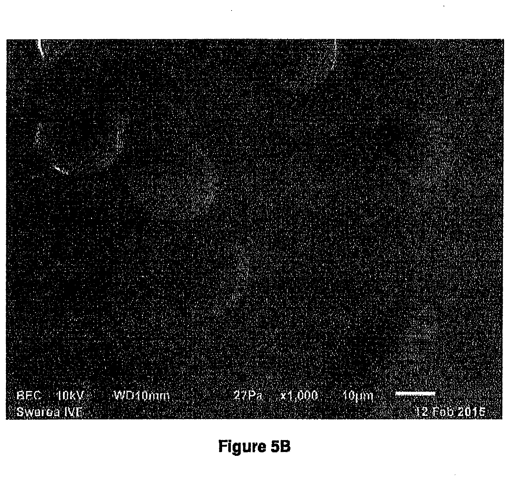

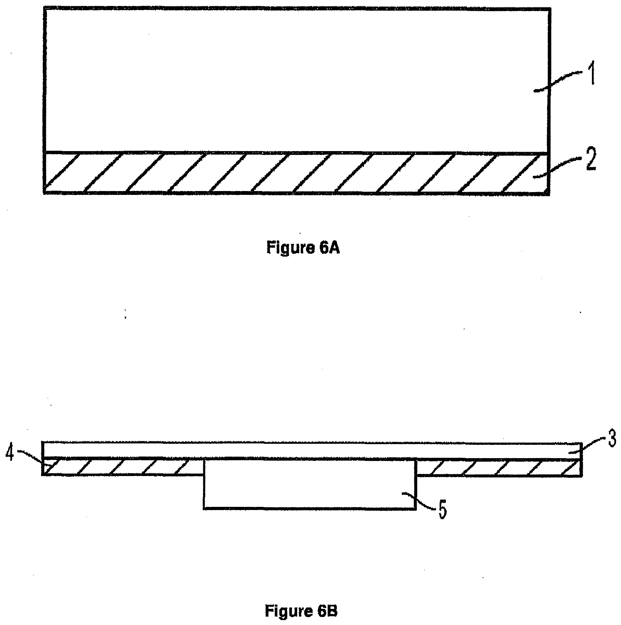

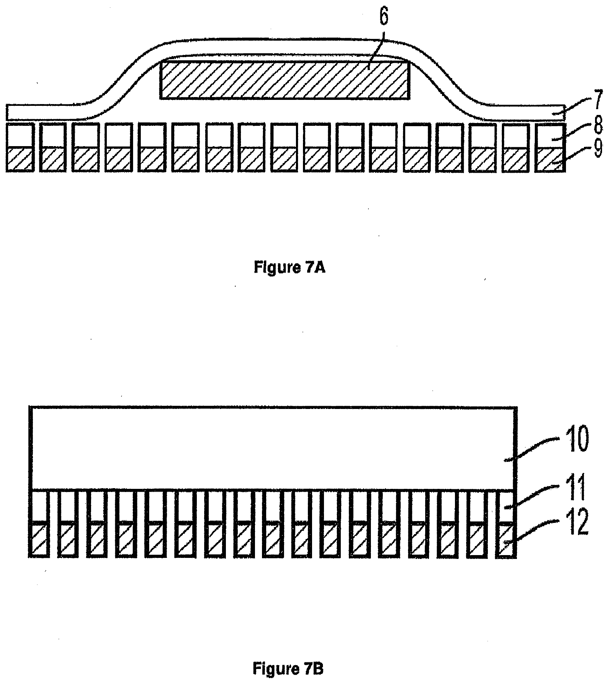

Certain non-limiting examples of wound dressings according to embodiments of the present disclosure are depicted In Figures 6A, 6B, 7A, and 7B. As described generally above, wound dressings of the present disclosure comprise a substrate (e.g. 1 in Figure 6A, **5** in Figure 6B, **6** In Figure 7A, and **10** in Figure 7B). In some embodiments, the wound dressing further comprises an adhesive layer (e.g. **2** in Figure 6A, **4** in Figure 6B, **9** In Figure 7A, and **12** In Figure 7B) for adhering to skin. In some embodiments, the adhesive layer is located on the bottom surface of the substrate. In some embodiments, the wound dressing further comprises a perforated film layer (e.g. **8** In Figure 7A, **11** in Figure 7B). In some embodiments, the perforated film layer is located between the substrate and the adhesive layer. In some embodiments, the wound dressing further comprises a backing layer (e.g. **3** in Figure 6B, **7** in Figure 7A). In some embodiments, wound dressings according to the present disclosure are used in combination with a secondary dressing that is applied on top of the wound dressing according to the disclosure

### B. Antimicrobial Coating Compositions

As described generally above, the present invention provides an antimicrobial coating composition for coating a wound dressing substrate, said composition comprising: an antimicrobial agent, one or more polymers, wherein the one or more polymers in the antimicrobial coating composition are selected from the group consisting of cellulosic polymers, neutral poly(meth)acrylate esters, polyvinylpyrrolidone, polyvinylpolypyrrolidone, and combinations thereof, a solvent system comprising a non-aqueous solvent, wherein the antimicrobial coating composition comprises 1-20% w/w of water, and wherein the non-aqueous solvent comprises a polar protic solvent. In general the antimicrobial coating composition comprises an antimicrobial agent and one or more polymers in a solvent system that comprises a non-aqueous solvent. The present disclosure also provides methods for producing an antimicrobial coating composition for coating a wound dressing substrate which comprises steps of mixing an antimicrobial agent and one or more polymers in a solvent system that comprises a non-aqueous solvent.

The term "antimicrobial coating composition" as used herein is distinguished from the term "antimicrobial coating" in that "antimicrobial coating" refers to the mixture of antimicrobial agent and one or more polymers that have been applied to the substrate and dried. Thus, the antimicrobial coating can be thought of as the residue that remains on the substrate after the antimicrobial coating composition is applied to the substrate and the substrate is dried to remove the solvents.

### Antimicrobial Agent

In some embodiments, the antimicrobial agent comprises silver. In some embodiments, the silver is metallic silver. In some embodiments, the silver is a silver salt. In some embodiments, the silver salt Is silver sulfate, silver chloride, silver nitrate, silver sulfadiazine, silver carbonate, silver phosphate, silver lactate, silver bromide, silver acetate, silver citrate, silver CMC, silver oxide. In some embodiments, the silver salt is silver sulfate.

In some embodiments, the antimicrobial agent comprises iodine. In some embodiments, the iodine is povidone iodine, cadexomer iodine, triocyn, or iodozyme.

In some embodiments, the antimicrobial agent comprises a monoguanide or biguanide. In some embodiments the monoguanide or biguanide is chlorhexidine digluconate, chlorhexidine diacetate, chlorhexidine dihydrochloride, polyhexamethylene-biguanide (PHMB) or a salt thereof, or polyhexamethylenemonoguanide (PHMG) or a salt thereof. In some embodiments the blguanlde is PHMB or a salt thereof.

In some embodiments, the antimicrobial agent comprises a quaternary ammonium compound. In some embodiments, the quaternary ammonium compound is cetylpyridinium chloride, benzethonium chloride, or poly-DADMAC.

In some embodiments, the antimicrobial agent comprises triclosan, sodium hypochlorite, copper, hydrogen peroxide, xylitol, or honey.

In some embodiments, the antimicrobial agent in the antimicrobial coating composition is present In an amount less than 40% w/w. In some embodiments, the antimicrobial agent in the antimicrobial coating composition is present in an amount less than 35% w/w. In some embodiments, the antimicrobial agent in the antimicrobial coating composition is present in an amount less than 30% w/w. In some embodiments, the antimicrobial agent in the antimicrobial coating composition is present in an amount less than 25% w/w. In some embodiments, the antimicrobial agent in the antimicrobial coating composition is present In an amount less than 20% w/w. In some embodiments, the antimicrobial agent in the antimicrobial coating composition is present In an amount less than 15% w/w. In some embodiments, the antimicrobial agent in the antimicrobial coating composition is present in an amount less than 10% w/w. In some embodiments, the antimicrobial agent in the antimicrobial coating composition is present in an amount less than 5% w/w.

In some embodiments, the antimicrobial agent in the antimicrobial coating composition is present in an amount from 0.1%t to 40% w/w. In some embodiments, the antimicrobial agent In the antimicrobial coating composition is present in an amount from 0.1% to 35%. In some embodiments, the antimicrobial agent In the antimicrobial coating composition is present in an amount from 0.1% to 30%. In some embodiments, the antimicrobial agent in the antimicrobial coating composition is present in an amount from 0.1 % to 25%. In some embodiments, the antimicrobial agent In the antimicrobial coating composition is present in an amount from 0.1% to 20%. In some embodiments, the antimicrobial agent In the antimicrobial coating composition is present in an amount from 0.1 % to 15%. In some embodiments, the antimicrobial agent in the antimicrobial coating composition is present in an amount from 0.1% to 10%. In some embodiments, the antimicrobial agent In the antimicrobial coating composition is present in an amount from 0.1% to 5%. In some embodiments, the antimicrobial agent In the antimicrobial coating composition is present in an amount from 0.1% to 1%. In some embodiments, the antimicrobial agent in the antimicrobial coating composition is present In an amount from 0.5% to 3%. In some embodiments, the antimicrobial agent in the antimicrobial coating composition is present In an amount from 1% to 40%. In some embodiments, the antimicrobial agent In the antimicrobial coating composition is present in an amount from 5% to 40%. In some embodiments, the antimicrobial agent in the antimicrobial coating composition is present in an amount from 10% to 40%. In some embodiments, the antimicrobial agent in the antimicrobial coating composition is present in an amount from 15% to 40%. In some embodiments, the antimicrobial agent in the antimicrobial coating composition Is present in an amount from 20% to 40%. In some embodiments, the antimicrobial agent in the antimicrobial coating composition is present in an amount from 25% to 40%. In some embodiments, the antimicrobial agent In the antimicrobial coating composition is present In an amount from 30% to 40%. In some embodiments, the antimicrobial agent In the antimicrobial coating composition Is present in an amount from 35% to 40%.

In some embodiments, the antimicrobial agent in the wound dressing substrate is present in an amount less than 30 mg/cm². In some embodiments, the antimicrobial agent In the wound dressing substrate is present In an amount less than 25 mg/cm². In some embodiments, the antimicrobial agent in the wound dressing substrate is present in an amount less than 20 mg/cm². in some embodiments, the antimicrobial agent in the wound dressing substrate is present in an amount less than 15 mg/cm². In some embodiments, the antimicrobial agent in the wound dressing substrate is present in an amount less than 10 mg/cm². In some embodiments, the antimicrobial agent in the wound dressing substrate is present in an amount less than 5 mg/cm². In some embodiments, the antimicrobial agent in the wound dressing substrate is present in an amount less than 1 mg/cm². in some embodiments, the antimicrobial agent In the wound dressing substrate is present in an amount less than 0.5 mg/cm².

In some embodiments, the antimicrobial agent in the wound dressing substrate is present in an amount from 0.01 mg/cm² to 30 mg/cm². In some embodiments, the antimicrobial agent in the wound dressing substrate is present in an amount from 0.01 mg/cm² to 35 mg/cm². In some embodiments, the antimicrobial agent in the wound dressing substrate is present in an amount from 0.01 mg/cm² to 30 mg/cm². In some embodiments, the antimicrobial agent in the wound dressing substrate is present in an amount from 0.01 mg/cm² to 25 mg/cm². In some embodiments, the antimicrobial agent In the wound dressing substrate is present In an amount from 0.01 mg/cm² to 20 mg/cm². In some embodiments, the antimicrobial agent in the wound dressing substrate is present in an amount from 0.01 mg/cm² to 15 mg/cm². In some embodiments, the antimicrobial agent in the wound dressing substrate is present in an amount from 0.01 mg/cm² to 10 mg/cm². In some embodiments, the antimicrobial agent in the wound dressing substrate is present in an amount from 0.01 mg/cm² to 5 mg/cm². In some embodiments, the antimicrobial agent in the wound dressing substrate is present In an amount from 0.05 mg/cm² to 3 mg/cm². in some embodiments, the antimicrobial agent in the wound dressing substrate Is present In an amount from 0.05 mg/cm² to 1 mg/cm². In some embodiments, the antimicrobial agent In the wound dressing substrate Is present In an amount from 0.1 mg/cm² to 1 mg/cm², for example, from 0.1 mg/cm² to 0.5 mg/cm². In some embodiments, the antimicrobial agent in the wound dressing substrate Is present in an amount from 0.1 mg/cm² to 40 mg/cm². In some embodiments, the antimicrobial agent In the wound dressing substrate is present in an amount from 0.5 mg/cm² to 40 mg/cm². In some embodiments, the antimicrobial agent in the wound dressing substrate is present in an amount from 1 mg/cm² to 40 mg/cm². In some embodiments, the antimicrobial agent in the wound dressing substrate Is present in an amount from 5 mg/cm² to 40 mg/cm². In some embodiments, the antimicrobial agent in the wound dressing substrate is present in an amount from 10 mg/cm² to 40 mg/cm². In some embodiments, the antimicrobial agent in the wound dressing substrate Is present in an amount from 15 mg/cm² to 40 mg/cm². In some embodiments, the antimicrobial agent In the wound dressing substrate is present in an amount from 20 mg/cm² to 40 mg/cm². In some embodiments, the antimicrobial agent in the wound dressing substrate Is present In an amount from 25 mg/cm² to 40 mg/cm². in some embodiments, the antimicrobial agent in the wound dressing substrate is present in an amount from 30 mg/cm² to 40 mg/cm². In some embodiments, the antimicrobial agent In the wound dressing substrate Is present in an amount from 35 mg/cm² to 40 mg/cm².

### Polymers

The one or more polymers in the antimicrobial coating composition are selected from the group consisting of cellulosic polymers, neutral poly(meth)acrylate esters, polyvinylpyrrolidone, polyvinylpolypyrrolidone, and combinations thereof.

In some embodiments, the one or more polymers In the coating composition are selected from cellulosic polymers. In some embodiments, the one or more polymers in the coating composition are cellulosic polymers selected from hydroxypropylmethylcellulose (HPMC), hydroxypropylcellulose (HPC), methylcellulose (MC), ethylcellulose (EC), and combinations thereof. In some embodiments, one of the one or more polymers in the coating composition is hydroxypropylcellulose. In some embodiments, two of the polymers in the coating composition are hydroxypropylcellulose and ethylcellulose.

In some embodiments, at least one of the one or more polymers in the antimicrobial coating composition is a neutral poly(meth)acrylate ester. In some embodiments, at least one of the one or more polymers in the coating composition is a methyl methacrylate / ethyl acrylate copolymers (e.g., a EUDRAGIT polymer).

In some embodiments, at least one of the one or more polymers In the antimicrobial coating composition is water soluble. As used herein, a "water soluble polymer" is soluble in water at 25 degrees Celsius at a concentration of at least 1 grams per liter. As used herein, "a non-water soluble polymer" is soluble in water at 25 degrees Celsius at a concentration of no more than 1 grams per liter. In some embodiments, the one or more polymers in the antimicrobial coating composition comprise a mixture of at least one water soluble polymer and at least one non-water soluble polymer.

In some embodiments, each of the one or more polymers in the antimicrobial coating composition is present In an amount less than 30% w/w. In some embodiments, each of the one or more polymers in the antimicrobial coating composition is present in an amount less than 20% w/w. In some embodiments, each of the one or more polymers in the antimicrobial coating composition is present In an amount less than 10% w/w. In some embodiments, each of the one or more polymers in the antimicrobial coating composition is present in an amount less than 5% w/w. In some embodiments, each of the one or more polymers In the antimicrobial coating composition is present In an amount less than 4% w/w. In some embodiments, each of the one or more polymers In the antimicrobial coating composition is present In an amount less than 3% w/w. In some embodiments, each of the one or more polymers in the antimicrobial coating composition is present in an amount less than 2% w/w. In some embodiments, each of the one or more polymers In the antimicrobial coating composition is present in an amount less than 1% w/w.

In some embodiments, each of the one or more polymers in the antimicrobial coating composition is present In an amount from 0.5% to 30% w/w. In some embodiments, each of the one or more polymers in the antimicrobial coating composition Is present in an amount from 0.5% to 20% w/w. In some embodiments, each of the one or more polymers in the antimicrobial coating composition is present In an amount from 0.5% to 10% w/w. In some embodiments, each of the one or more polymers in the antimicrobial coating composition is present In an amount from 0.5% to 5% w/w. In some embodiments, each of the one or more polymers In the antimicrobial coating composition is present in an amount from 0.5% to 4% w/w. In some embodiments, each of the one or more polymers In the antimicrobial coating composition is present in an amount from 0.5% to 3% w/w. In some embodiments, each of the one or more polymers In the antimicrobial coating composition is present In an amount from 1% to 2% w/w.

In some embodiments, the one or more polymers in the antimicrobial coating composition have an average molecular weight from 50 - 1,500 kDa. In some embodiments, the one or more polymers in the antimicrobial coating composition have an average molecular weight from 300 - 1,500 kDa. In some embodiments, the one or more polymers In the antimicrobial coating composition have an average molecular weight from 500 - 1,500 kDa. In some embodiments, the one or more polymers In the antimicrobial coating composition have an average molecular weight from 800 - 1,500 kDa. In some embodiments, the one or more polymers in the antimicrobial coating composition have an average molecular weight from 800 - 900 kDa. In some embodiments, the one or more polymers in the antimicrobial coating composition have an average molecular weight from 800 - 1,000 kDa. In some embodiments, the one or more polymers In the antimicrobial coating composition have an average molecular weight from 800 - 1,200 kDa. In some embodiments, the one or more polymers In the antimicrobial coating composition have an average molecular weight from 1,000 - 1,200 kDa. In some embodiments, the one or more polymers In the antimicrobial coating composition have an average molecular weight from 1,100 - 1,200 kDa.

### Non-Aqueous Solvent

The antimicrobial coating composition comprises a non-aqueous solvent. The non-aqueous solvent comprises a polar protic solvent. In some embodiments, the polar protic solvent comprises an alcohol. In some embodiments, the polar protic solvent solvent comprises a C₁₋₄ alkyl alcohol. In some embodiments, the polar protic solvent comprises methanol, ethanol, *n*-propanol, isopropanol, *n*-butanol, or *s*-butanol. In some embodiments, the polar protic solvent comprises ethanol.

The tho antimicrobial coating composition comprises from 1% to 20% w/w of water. In some embodiments, the antimicrobial coating composition comprises from 1% to 10% w/w of water. In some embodiments, the antimicrobial coating composition comprises from 10% to 20% w/w of water.

### Methods for Preparing Antimicrobial Coating Compositions

In some embodiments, the present disclosure provides methods for producing an antimicrobial coating composition for coating a wound dressing substrate which comprises steps of mixing an antimicrobial agent and one or more polymers in a solvent system that comprises a non-aqueous solvent. Suitable antimicrobial agents, polymers and non-aqueous solvents include those described in embodiments herein.

In some embodiments, the process for preparing an antimicrobial coating composition comprising the steps of : (1) adding one or more polymers to a solvent system that comprises a non-aqueous solvent; and (2) adding an antimicrobial agent to the mixture resulting from step (1).

In some embodiments, (1) a mixture of the one or more polymers in water is added to a non-aqueous solvent; and (2) a mixture of the antimicrobial agent in a non-aqueous solvent Is added to the mixture resulting from step (1). In some embodiments, the water in step (1) is heated. In some embodiments, the water in step (1) is heated such that the mixture in step (1) is a suspension of the one and more polymers in water. In some embodiments the non-aqueous solvent in steps (1) and (2) is the same.

In some embodiments, a mixture of the one or more polymers and the antimicrobial agent in water is added to the non-aqueous solvent. In some embodiments, the water is heated such that the mixture is a suspension.

In some embodiments, the one or more polymers are added to a mixture of the antimicrobial agent in the solvent system that comprises a non-aqueous solvent. In some embodiments, the one or more polymers are added to a mixture of the antimicrobial agent in a non-aqueous solvent.

In some embodiments, (1) the one or more polymers are added to the non-aqueous solvent; and (2) the antimicrobial agent is added to the mixture resulting from step (1).

In some embodiments, a mixture of the antimicrobial agent In a non-aqueous solvent is added to a mixture of the one or more polymers in a non-aqueous solvent. In some embodiments the non-aqueous solvent In both solutions is the same.

In some embodiments, the water In any of these methods is heated to from 40 to 70 degrees Celsius. In some embodiments, the water is heated to 60 degrees Celsius.

In some embodiments, the process further comprises the step of allowing the mixture to increase In viscosity. In some embodiments, the viscosity of the mixture is increased by mixing for at least 10, 20, 30, 40, 50 or 60 minutes. In some embodiments, the viscosity of the coating composition is at least 100 mPa·s ( 100 centipoise ) when measured according to the viscosity method disclosed below (see Example 7). In some embodiments, the viscosity of the coating composition is from 100 to 100000 mPa·s ( 100 to 100000 centipoise), for example, from 5000 to 50000 mPa·s (5000 to 50000 centipoise), such as from 5000 to 30000 mPa·s (5000 to 30000 centipoise ) or from 10000 to 20000 mPa·s (10000 to 20000 centipoise), when measured according to the viscosity method disclosed below (see Example 7).

### II. Absorbent antimicrobial wound dressing

In another aspect, the present disclosure provides an absorbent antimicrobial wound dressing prepared by a method which comprises steps of (a) preparing an antimicrobial coating composition by mixing an antimicrobial agent and one or more polymers in a solvent system that comprises a non-aqueous solvent, wherein the one or more polymers in the antimicrobial coating composition are selected from the group consisting of cellulosic polymers, neutral poly(meth)acrylate esters, polyvinylpyrrolidone, polyvinylpolypyrrolidone, and combinations thereof, wherein the antimicrobial coating composition comprises 1-20% w/w of water, (b) contacting the antimicrobial coating composition of step (a) with a wound dressing substrate which comprises absorbent fibers wherein the wound dressing substrate comprises cross-linked absorbent fibers, wherein the cross-linked absorbent fibers are capable of forming a swollen coherent gel upon absorbing a liquid, and (c) drying the product of step (b).

In yet another aspect, the present diclosure provides an absorbent antimicrobial wound dressing that includes a substrate comprising an absorbent fiber or absorbent particle coated with an antimicrobial coating that comprises an antimicrobial agent and one or more polymers, wherein the one or more polymers are selected from the group consisting of cellulosic polymers, neutral poly(meth)acrylate esters, polyvinylpyrrolidone; polyvinylpolypyrrolidone, and combinations thereof. As used herein, the terms "absorbent fiber or absorbent particle coated with an antimicrobial coating" denotes an absorbent fiber or absorbent particle which has some amount of an antimicrobial coating associated with at least a portion of its surface. It does not require complete or uniform coating of an absorbent fiber or absorbent particle. In fact, in some embodiments, a wound dressing substrate of the present disclosure may Include a mixture of (a) absorbent fibers (or absorbent particles) with no amount of antimicrobial coating on their surface and (b) absorbent fibers (or absorbent particles) with portions of their surface associated with an antimicrobial coating. Scanning electron microscope images of some exemplary wound dressing substrates of the present disclosure are provided In Figures 5A and 5B to illustrate this.

As shown in Figure 5B, the antimicrobial agents of the wound dressings of the present disclosure, here embodied by silver particles displayed in white, do not penetrate within the fibers of the substrate, but rather are solely located on the surface of the fibers.

### EXAMPLES

### Example 1. Preparation of Antimicrobial Coating Composition.

Silver sulfate (3.6 grams; commercially available from Sigma-Aldrich) was added to 190 proof ethanol (of about 100 grams of total 300 grams) in a beaker after which the mixture was agitated at 11000 rpm for 10 minutes with a rotor/stator mixer. Water (35 grams, 10%w/w of total weight of the total mixture) at 65 degrees Celsius was added to hydroxypropyl cellulose (HPC, 4.3 grams; commercially available from Ashland) in a beaker and swirled around for a few seconds until an even mixture was obtained. The beaker was immediately mounted under an overhead stirrer equipped with a dissolver blade after which the stirrer was started. The ethanol/silver sulfate mixture was then carefully added to the beaker during mixing. The silver sulfate residuals were rinsed out into the beaker using the rest of the ethanol (approximately 200 grams). The combined ethanol/water/sliver sulfate/HPC mixture was mixed for at least 60 minutes, increasing the mixing speed gradually from 50 to 2000 rpm as viscosity Increased.

### Example 2. Application to Substrate and Drying

The antimicrobial coating composition from Example 1 is coated onto a siliconized release paper (POLY SLIK^{®} commercially available from Loparex). A substrate of non-woven (cross-linked PVA fibers; 250 gsm) (Exufiber^{®} commercially available from Mölnlycke Health Care) was pressed against the antimicrobial coating on the release layer using a roller weight (2,2 kg) so that the antimicrobial coating was transferred into the non-woven substrate. After this the non-woven substrate was removed from the release paper and transferred onto a hot plate (80°C) and dried for 2 minutes with the dry side facing the hotplate. The same procedure was then repeated for the other side of the non-woven substrate so that the product had been coated on both sides.

### Example 3. Prototypes for testing

A number of prototypes, 1 to 8 as presented in Table 1 below, were prepared. Prototype 2 was prepared according to Example 1 followed by Example 2. Prototype 3 was prepared according to Example 1 and Example 2, but with the exceptions that different concentrations of HPC and/or silver sulfate were used as listed in Table 1. Prototype 1 Including a silver coating with no HPC, was prepared by first preparing a silver coating composition according to Example 1 but with no added HPC, and subsequently the non-woven substrate (same as in Example 2) was dipped into the silver coating composition (consisting of a suspension of silver sulfate in ethanol), which silver coating composition was constantly stirred using a spatula in order to avoid sedimentation of the silver sulfate. The non-woven substrate was dried on a hot plate (80°C) for about 10 minutes, until dried. Prototypes 4, 5 and 6 were prepared according to Example 1 and Example 2, with the following exceptions (presented In Table 1): (i) a non-woven substrate (40 gsm) (Fibrella^{®} 2000 commercially available from Suominen Corporation, Helsinki Finland) was used in the preparation of Prototype 5; and a foam substrate (thickness of 1.5 mm) (Mepilex^{®} Transfer, commercially available from Mölnlycke Health Care) was used in the preparation of Prototype 6; (ii) different concentrations of HPC and sliver sulfate; and (iii) the silver coating composition was only applied to one side of the substrates of Prototypes 4, 5 and 6 (the non-adhesive foam side). In Prototypes 7 and 8, the silver sulfate coating composition prepared according to Example 1, with HPC and silver sulfate concentrations as specified In Table 1, was applied on the substrates using slot die coating, and subsequently dried in a hot air convection oven. It should be noted that the HPC concentrations listed In Table 1 refers to the concentration of HPC In the coating composition prepared according to Example 1, i.e. not the actual HPC concentration in the dried product. The silver (Ag⁺) amount per area unit as given in Table 1, was calculated by weighing the substrate before applying the silver coating composition and subsequently weighing the coated substrate (before drying thereof), thus the amount of silver coating composition picked up by the substrate can be calculated and the silver (Ag⁺) amount per area unit can be determined given a known silver concentration.

**Table 1**

| ***Prototype no.*** | ***HPC concentration (%w*/*w)*** | ***Silver sulfate concentration (%w*/*w)*** | ***Silver (Ag+) amount (mg*/*cm²)*** | ***Substrate*** |
|---|---|---|---|---|
| **1** | 0.00 | 1.00 | 0.12 | Exufiber^{®} |
| **2** | 1.25 | 1.05 | 0.12 | Exufiber^{®} |
| **3** | 2.25 | 1.04 | 0.12 | Exufiber^{®} |
| **4** | 1.25 | 1.10 | 0.06 | Exufiber^{®} |
| **5** | 1.25 | 1.10 | 0.06 | Fibrella^{®} 2000 |
| **6** | 1.25 | 1.10 | 0.06 | Mepilex^{®} Transfer |
| **7** | 1.21 | 2.30 | 0.13 | Exufiber^{®} |
| **8** | 1.21 | 2.30 | 0.23 | Exufiber^{®} |

### Example 4. Release of silver sulfate

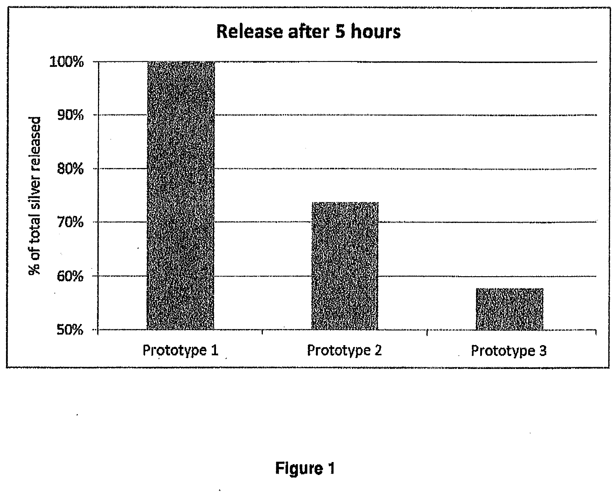

The release of sliver sulfate from prototypes 1 to 3, was measured by immerse a circular test piece (10 cm² radius) to a vessel of a USP bath containing deionized water (70 ml, 32 deg. C). Paddle rotation speed was set to 125 rpm and a 1 ml sample were extracted after 5 hours. The silver sulfate concentration in the extracted samples were analyzed using inductively coupled plasma optical emission spectroscopy (ICP-OES). The silver is determined at wavelengths 328.068 nm and 338.289 nm in axial mode, where 328.068 Is used for quantification and 338.289 nm is used to detect Interferences. Each sample is measured three times. Figure 1 shows the total amount of silver that is released in 5 hours. As can be seen In Figure 1, a non-woven substrate coated with a silver coating composition having a higher concentration of HPC (e.g. Prototype 3) has a lower total silver release as compared to Prototype 2 with less HPC, or Prototype 1 with no HPC. Accordingly, the results show that the release of silver may be controlled by adjusting the amount of HPC in the coating composition.

### Example 5. Reduced Discoloration of Wound Dressing Substrate

Prototypes 4 to 6 were tested for discoloration due to the presence of silver salt. Prototypes 4 to 6 (ca. 100-150 cm²) were subjected to a test environment of 55°C and 80%RH (Oven VC 0020 from Vötsch Industritechnik), to thereby accelerate the ageing process. Colour was measured (on the coated side of each Prototypes 4-6) at different time points according to ASTM D 2244 - 11, and the colour change (dE) was calculated compared to uncoated reference samples (not subjected to the test environment) corresponding to the respective substrate of each Prototype tested but without silver coating. Figure 4 shows colour change observed for the Prototypes 4 to 6. As can be seen In Figure 4, Prototype 4 comprising a non-woven PVA substrate (Exufiber^{®}) exhibited a much reduced colour change as compared to the Prototype 5 (Fibrella^{®} 2000 substrate) and Prototype 6 (Mepilex^{®} Transfer foam substrate).

### Example 6. Scanning Electron Images of Wound Dressing Substrate

Scanning electron micrograph images of the wound dressing substrates were obtained using a low vacuum SEM. Two types of detector are used, a Secondary Electron (SE)-detector (labeled LSEI = Low vacuum Secondary Electron Image) and a Backscattered Electron (BEC) detector (labeled BEC = Backscattered Electron Composition). Cross sections of fibers shown in Figure 5B are produced by vacuum impregnating a test piece of Prototype 8 In epoxy and then producing a smooth surface by grinding and ion polishing. In the picture of Figure 5B, heavy substances, such as silver, will be displayed in white.

### Example 7. Method of measuring viscosity

The viscosity of a test mixture, e.g. antimicrobial coating composition, is measured using a Brookfield Viscometer Instrument Model LVF. In case the molecular weight of the polymer, e.g. HPC, in the antimicrobial coating composition is known, Table 2 below provides a guidance as to which Brookfield viscometer spindle and spindle rotation to use. The viscosity of the test mixture (at 25°±0,2°C) is measured by inserting the appropriate Brookfield viscometer spindle Into the test mixture and then starting the spindle rotating. The test mixture is rotated for 3 minutes, and the instrument is stopped
before taking the reading. The reading was multiplied by the factor (as provided with the instrument) corresponding to the speed and spindle used. The result is the viscosity of the test mixture in mPa^{·}s (centipoise).

**Table 2**

| ***Molecular weight (kDa) of polymer*** | ***Brookfield settings*** | |
|---|---|---|
| | **rpm** | **Spindle No.** |
| < 87,500 | 30 | 2 |
| 87,500-117,500 | 30 | 1 |
| 117,501-255,000 | 60 | 2 |
| 255,001-610,000 | 60 | 2 |
| 610,001-1,000,000 | 60 | 4 |
| > 1,000,000 | 30 | 3 |

## Claims

1. An antimicrobial coating composition for coating a wound dressing substrate, said composition comprising:
• an antimicrobial agent,
• one or more polymers, wherein the one or more polymers in the antimicrobial coating composition are selected from the group consisting of cellulosic polymers, neutral poly(meth)acrylate esters, polyvinylpyrrolidone, polyvinylpolypyrrolidone, and combinations thereof,
• a solvent system comprising a non-aqueous solvent,
wherein the antimicrobial coating composition comprises 1-20% w/w of water, and wherein the non-aqueous solvent comprises a polar protic solvent.

2. The antimicrobial coating composition according to claim 1, wherein
(i) the antimicrobial agent comprises silver, wherein the silver is preferably metallic silver or a silver salt, wherein the silver salt is preferably silver sulfate, silver chloride, silver nitrate, silver sulfadiazine, silver carbonate, silver phosphate, silver lactate, silver bromide, silver acetate, silver citrate, silver CMC, or silver oxide, or
(ii) the antimicrobial agent comprises iodine, wherein the iodine is preferably povidone iodine, cadexomer iodine, or iodozyme, or
(iii) the antimicrobial agent comprises a monoguanide or biguanide, wherein the monoguanide or biguanide is chlorhexidine digluconate, chlorhexidine diacetate, chlorhexidine dihydrochloride, polyhexamethylene-biguanide (PHMB) or a salt thereof, or polyhexamethylenemonoguanide (PHMG) or a salt thereof, or
(iv) the antimicrobial agent comprises a quaternary ammonium compound, wherein the quaternary ammonium compound preferably is cetylpyridinium chloride, benzethonium chloride, or poly-DADMAC, or
(v) the antimicrobial agent comprises triclosan, sodium hypochlorite, copper, hydrogen peroxide, xylitol, or honey.

3. The antimicrobial coating composition according to claim 1 or claim 2, wherein the antimicrobial agent is present in an amount of less than 40% w/w.

4. The antimicrobial coating composition according to any one of the preceding claims, wherein each of the one or more polymers in the antimicrobial coating composition is present in an amount less than 30% w/w.

5. The antimicrobial coating composition according to any one of the preceding claims, wherein the polar protic solvent comprises an alcohol, preferably a C₁₋₄ alcohol, more preferably methanol, ethanol, *n*-propanol, isopropanol, n-butanol, or s-butanol, even more preferably ethanol.

6. The antimicrobial coating composition according to any one of the preceding claims, wherein the one or more polymers in the antimicrobial coating composition are cellulosic polymers selected from the group consisting of hydroxypropylmethylcellulose (HPMC), hydroxypropylcellulose (HPC), methylcellulose (MC), and ethylcellulose (EC), preferably hydroxypropylcellulose (HPC).

7. The antimicrobial coating composition according to any one of the preceding claims, wherein the antimicrobial coating composition comprises 10-20% w/w of water.

8. A method of preparing an absorbent antimicrobial wound dressing comprising:
(a) preparing an antimicrobial coating composition by mixing an antimicrobial agent and one or more polymers in a solvent system that comprises a non-aqueous solvent, wherein the one or more polymers in the antimicrobial coating composition are selected from the group consisting of cellulosic polymers, neutral poly(meth)acrylate esters, polyvinylpyrrolidone, polyvinylpolypyrrolidone, and combinations thereof, wherein the antimicrobial coating composition comprises 1-20% w/w of water;
(b) contacting the antimicrobial coating composition of step (a) with a wound dressing substrate which comprises absorbent fibers, wherein the wound dressing substrate comprises cross-linked absorbent fibers, wherein the cross-linked absorbent fibers are capable of forming a swollen coherent gel upon absorbing a liquid; and
(c) drying the product of step (b).

9. The method according to claim 8 , wherein the antimicrobial agent comprises silver, preferably silver oxide or a silver salt.

10. The method according to any one of claims 8 to 9, wherein the non-aqueous solvent comprises a polar protic solvent, preferably an alcohol, wherein the alcohol preferably comprises a C₁₋₄ alkyl alcohol, more preferably methanol, ethanol, *n*-propanol, isopropanol, *n*-butanol, or *s*-butanol, even more preferably ethanol.

11. The method according to any one of claims 8-10, wherein the one or more polymers in the antimicrobial coating composition are cellulosic polymers selected from the group consisting of hydroxypropylmethylcellulose (HPMC), hydroxypropylcellulose (HPC), methylcellulose (MC), and ethylcellulose (EC), preferably hydroxypropylcellulose (HPC).

12. The method according to any one of of claims 8-11 wherein the antimicrobial coating composition comprises 10-20% w/w of water.

## Patentansprüche

1. Antimikrobielle Beschichtungszusammensetzung zum Beschichten eines Wundverbandsubstrats, wobei die Zusammensetzung Folgendes umfasst:
- ein antimikrobielles Mittel,
- ein oder mehrere Polymere, wobei das eine oder die mehreren Polymere in der antimikrobiellen Beschichtungszusammensetzung ausgewählt sind aus der Gruppe bestehend aus Zellulosepolymeren, neutralen Poly(meth)acrylatestern, Polyvinylpyrrolidon, Polyvinylpolypyrrolidon und Kombinationen davon,
- ein Lösemittelsystem, das ein nicht-wässriges Lösemittel umfasst,
wobei die antimikrobielle Beschichtungszusammensetzung 1-20 Gew.-% Wasser umfasst und das nicht-wässrige Lösungsmittel ein polares protisches Lösungsmittel umfasst.

2. Antimikrobielle Beschichtungszusammensetzung nach Anspruch 1, wobei
(i) das antimikrobielle Mittel Silber umfasst, wobei das Silber vorzugsweise metallisches Silber oder ein Silbersalz ist, wobei das Silbersalz vorzugsweise Silbersulfat, Silberchlorid, Silbernitrat, Silbersulfadiazin, Silbercarbonat, Silberphosphat, Silberlactat, Silberbromid, Silberacetat, Silbercitrat, Silber-CMC oder Silberoxid ist, oder
(ii) das antimikrobielle Mittel lod umfasst, wobei das lod vorzugsweise Povidonlod, Cadexomer-Iod oder lodozym ist, oder
(iii) das antimikrobielle Mittel ein Monoguanid oder Biguanid umfasst, wobei das Monoguanid oder Biguanid Chlorhexidindigluconat, Chlorhexidindiacetat, Chlorhexidindihydrochlorid, Polyhexamethylenbiguanid (PHMB) oder ein Salz davon ist, oder Polyhexamethylenmonoguanid (PHMG) oder ein Salz davon ist, oder
(iv) das antimikrobielle Mittel eine quaternäre Ammoniumverbindung umfasst, wobei die quaternäre Ammoniumverbindung vorzugsweise Cetylpyridiniumchlorid, Benzethoniumchlorid oder Poly-DADMAC ist, oder
(v) das antimikrobielle Mittel Triclosan, Natriumhypochlorit, Kupfer, Wasserstoffperoxid, Xylit oder Honig umfasst.

3. Antimikrobielle Beschichtungszusammensetzung nach Anspruch 1 oder Anspruch 2, wobei das antimikrobielle Mittel in einer Menge von weniger als 40 Gew.-% vorhanden ist.

4. Antimikrobielle Beschichtungszusammensetzung nach einem der vorhergehenden Ansprüche, wobei jedes der ein oder mehreren Polymere in der antimikrobiellen Beschichtungszusammensetzung in einer Menge von weniger als 30 Gew.-% vorhanden ist.

5. Antimikrobielle Beschichtungszusammensetzung nach einem der vorhergehenden Ansprüche, wobei das polare protische Lösungsmittel einen Alkohol, vorzugsweise einen C₁₋₄-Alkohol, besonders bevorzugt Methanol, Ethanol, n-Propanol, Isopropanol, n-Butanol oder s-Butanol, noch mehr bevorzugt Ethanol, umfasst.

6. Antimikrobielle Beschichtungszusammensetzung nach einem der vorhergehenden Ansprüche, wobei das eine oder die mehreren Polymere in der antimikrobiellen Beschichtungszusammensetzung Cellulosepolymere sind, ausgewählt aus der Gruppe bestehend aus Hydroxypropylmethylcellulose (HPMC), Hydroxypropylcellulose (HPC), Methylcellulose (MC) und Ethylcellulose (EC), vorzugsweise Hydroxypropylcellulose (HPC).

7. Antimikrobielle Beschichtungszusammensetzung nach einem der vorhergehenden Ansprüche, wobei die antimikrobielle Beschichtungszusammensetzung 10-20 Gew.-% Wasser umfasst.

8. Verfahren zur Herstellung eines absorbierenden antimikrobiellen Wundverbands, umfassend:
(a) Herstellen einer antimikrobiellen Beschichtungszusammensetzung durch Mischen eines antimikrobiellen Mittels und eines oder mehrerer Polymere in einem Lösungsmittelsystem, das ein nicht-wässriges Lösungsmittel umfasst, wobei das eine oder die mehreren Polymere in der antimikrobiellen Beschichtungszusammensetzung ausgewählt sind aus der Gruppe bestehend aus Cellulosepolymeren, neutralen Poly(meth)acrylatestern, Polyvinylpyrrolidon, Polyvinylpolypyrrolidon und Kombinationen davon, wobei die antimikrobielle Beschichtungszusammensetzung 1-20 Gew.-% Wasser umfasst;
(b) Inkontaktbringen der antimikrobiellen Beschichtungszusammensetzung von Schritt (a) mit einem Wundverbandsubstrat, das absorbierende Fasern umfasst, wobei das Wundverbandsubstrat vernetzte absorbierende Fasern umfasst, wobei die vernetzten absorbierenden Fasern in der Lage sind, ein gequollenes kohärentes Gel zu bilden, wenn sie eine Flüssigkeit absorbieren; und
(c) Trocknen des Produkts von Schritt (b).

9. Verfahren nach Anspruch 8, wobei das antimikrobielle Mittel Silber, vorzugsweise Silberoxid oder ein Silbersalz, umfasst..

10. Verfahren nach einem der Ansprüche 8 bis 9, wobei das nicht-wässrige Lösungsmittel ein polares protisches Lösungsmittel, vorzugsweise einen Alkohol, umfasst, wobei der Alkohol vorzugsweise einen C₁₋₄-Alkylalkohol umfasst, besonders bevorzugt Methanol, Ethanol, n-Propanol, Isopropanol, n-Butanol oder s-Butanol, noch mehr bevorzugt Ethanol.

11. Verfahren nach einem der Ansprüche 8-10, wobei das eine oder die mehreren Polymere in der antimikrobiellen Beschichtungszusammensetzung Cellulosepolymere sind, die aus der Gruppe ausgewählt sind, die aus Hydroxypropylmethylcellulose (HPMC), Hydroxypropylcellulose (HPC), Methylcellulose (MC) und Ethylcellulose (EC), vorzugsweise Hydroxypropylcellulose (HPC), besteht.

12. Verfahren nach einem der Ansprüche 8-11, wobei die antimikrobielle Beschichtungszusammensetzung 10-20 Gew.-% Wasser umfasst.

## Revendications

1. Composition de revêtement antimicrobien pour revêtir un substrat de pansement, ladite composition comprenant :
• un agent antimicrobien,
• un ou plusieurs polymères, dans laquelle les un ou plusieurs polymères dans la composition de revêtement antimicrobien sont choisis parmi le groupe constitué de polymères cellulosiques, d'esters poly(méth)acrylate neutres, de polyvinylpyrrolidone, de polyvinylpolypyrrolidone et de combinaisons de ceux-ci,
• un système de solvant comprenant un solvant non aqueux,
dans laquelle la composition de revêtement antimicrobien comprend de 1 à 20 % p/p d'eau, et dans laquelle le solvant non aqueux comprend un solvant protique polaire.

2. Composition de revêtement antimicrobien selon la revendication 1, dans laquelle
(i) l'agent antimicrobien comprend de l'argent, dans laquelle l'argent est préférentiellement de l'argent métallique ou un sel d'argent, dans laquelle le sel d'argent est préférentiellement le sulfate d'argent, le chlorure d'argent, le nitrate d'argent, la sulfadiazine d'argent, le carbonate d'argent, le phosphate d'argent, le lactate d'argent, le bromure d'argent, l'acétate d'argent, le citrate d'argent, la CMC d'argent, ou l'oxyde d'argent, ou
(ii) l'agent antimicrobien comprend de l'iode, dans laquelle l'iode est préférentiellement de la povidone iodée, du cadexomère iodé, ou de l'iodozyme, ou
(iii) l'agent antimicrobien comprend un monoguanide ou un biguanide, dans laquelle le monoguanide ou le biguanide est le digluconate de chlorhexidine, le diacétate de chlorhexidine, le dichlorhydrate de chlorhexidine, le polyhexaméthylène-biguanide (PHMB) ou un sel de celui-ci, ou le polyhexaméthylènemonoguanide (PHMG) ou un sel de celui-ci, ou
(iv) l'agent antimicrobien comprend un composé d'ammonium quaternaire, dans laquelle le composé d'ammonium quaternaire est préférentiellement le chlorure de cétylpyridinium, le chlorure de benzéthonium ou le poly-DADMAC, ou
(v) l'agent antimicrobien comprend du triclosan, de l'hypochlorite de sodium, du cuivre, du peroxyde d'hydrogène, du xylitol ou du miel.

3. Composition de revêtement antimicrobien selon la revendication 1 ou la revendication 2, dans laquelle l'agent antimicrobien est présent en une quantité inférieure à 40 % p/p.

4. Composition de revêtement antimicrobien selon l'une quelconque des revendications précédentes, dans laquelle chacun des un ou plusieurs polymères dans la composition de revêtement antimicrobien est présent en une quantité inférieure à 30 % p/p.

5. Composition de revêtement antimicrobien selon l'une quelconque des revendications précédentes, dans laquelle le solvant protique polaire comprend un alcool, préférentiellement un alcool en C₁₋₄, plus préférentiellement le méthanol, l'éthanol, le *n-*propanol, l'isopropanol, le *n*-butanol ou le s-butanol, encore plus préférentiellement l'éthanol.

6. Composition de revêtement antimicrobien selon l'une quelconque des revendications précédentes, dans laquelle les un ou plusieurs polymères dans la composition de revêtement antimicrobien sont des polymères cellulosiques choisis parmi le groupe constitué d'hydroxypropylméthylcellulose (HPMC), d'hydroxypropylcellulose (HPC), de méthylcellulose (MC) et d'éthylcellulose (EC), préférentiellement d'hydroxypropylcellulose (HPC).

7. Composition de revêtement antimicrobien selon l'une quelconque des revendications précédentes, dans laquelle la composition de revêtement antimicrobien comprend de 10 à 20 % p/p d'eau.

8. Procédé de préparation d'un pansement antimicrobien absorbant comprenant :
(a) la préparation d'une composition de revêtement antimicrobien par mélange d'un agent antimicrobien et d'un ou plusieurs polymères dans un système de solvant qui comprend un solvant non aqueux, dans lequel les un ou plusieurs polymères dans la composition de revêtement antimicrobien sont choisis parmi le groupe constitué de polymères cellulosiques, d'esters poly(méth)acrylate neutres, de polyvinylpyrrolidone, de polyvinylpolypyrrolidone et de combinaisons de ceux-ci, dans lequel la composition de revêtement antimicrobien comprend de 1 à 20 % p/p d'eau ;
(b) la mise en contact de la composition de revêtement antimicrobien de l'étape (a) avec un substrat de pansement qui comprend des fibres absorbantes, dans lequel le substrat de pansement comprend des fibres absorbantes réticulées, dans lequel les fibres absorbantes réticulées sont aptes à former un gel cohérent gonflé lors de l'absorption d'un liquide ; et
(c) le séchage du produit de l'étape (b).

9. Procédé selon la revendication 8, dans lequel l'agent antimicrobien comprend de l'argent, préférentiellement de l'oxyde d'argent ou un sel d'argent.

10. Procédé selon l'une quelconque des revendications 8 à 9, dans lequel le solvant non aqueux comprend un solvant protique polaire, préférentiellement un alcool, dans lequel l'alcool comprend préférentiellement un alcool alkylique en C₁₋₄, plus préférentiellement le méthanol, l'éthanol, le n-propanol, l'isopropanol, le n-butanol ou le s-butanol, encore plus préférentiellement l'éthanol.

11. Procédé selon l'une quelconque des revendications 8 à 10, dans lequel les un ou plusieurs polymères dans la composition de revêtement antimicrobien sont des polymères cellulosiques choisis parmi le groupe constitué d'hydroxypropylméthylcellulose (HPMC), d'hydroxypropylcellulose (HPC), de méthylcellulose (MC) et d'éthylcellulose (EC), préférentiellement d'hydroxypropylcellulose (HPC).

12. Procédé selon l'une quelconque des revendications 8 à 11, dans lequel la composition de revêtement antimicrobien comprend de 10 à 20 % p/p d'eau.
